# EUROPEAN PATENT APPLICATION

(11) **EP 3 167 893 A1**
(43) Date of publication of application: **17.05.2017**
(21) Application number: 15806181.2
(22) Date of filing: 12.06.2015
(51) Int. Cl.: A61K 36/02, A61P 27/02, A61P 27/00

(54) **PHARMACEUTICAL COMPOSITION CONTAINING SPIRULINA MAXIMA EXTRACT AS ACTIVE INGREDIENT FOR PREVENTING AND TREATING RETINAL DISEASES**

(30) Priority: 13.06.2014 KR 20140071960
(71) Applicant: JCREATION, Jeju-si, Jeju-do 695-982 (KR); Jung, Woo Chang, Seoul 130-730 (KR)
(72) Inventor: CHOUNG, Se Young, Seoul 130-730 (KR); KIM, Dong Joon, Jeju-si Jeju-do 695-982 (KR); YANG, Seung Won, Yongin-si Gyeonggi-do 448-130 (KR); CHOI, Sang Ho, Suwon-si Gyeonggi-do 441-865 (KR); KANG, Do Hyung, Ansan-si Gyeonggi-do 426-744 (KR); HEO, Soo Jin, Ansan-si Gyeonggi-do 426-906 (KR)
(74) Representative: Coggins, Julia Frances
(86) International application number: PCT/KR2015/005952
(87) International publication number: WO 2015/190875

(57) **Abstract**

A Spirulina maxima extract of the present invention and Allophycocyanin (APC), R-phycoerythrin (R-PE), and C-phycocyanin (C-PC), which are components of the Spirulina maxima extract, show an effect of inhibiting cell death and A2E (pyridinium bis-retinoid), which is oxidized with a blue light, so that the present invention can be usefully applied as an active ingredient in a composition for preventing and treating retinal diseases.

## Description

### BACKGROUND OF THE INVENTION

### 1. Field of the Invention

The present invention relates to a pharmaceutical composition containing Spirulina maxima extract as an active ingredient for preventing and treating retinal diseases.

### 2. Description of the Related Art

People in this modern society who are living in the flood of visual culture and thus getting at least 80 ~ 90% of life information through eyes experience decreased visual acuity continuously, suggesting that the rate of decreased visual acuity caused by environmental reasons is continuously increasing. Modern people spend most of their time in front of a variety of digital screens such as computers and smart phones, etc. The problem is that LED used for the screen of such a digital device as the smart-phone emits blue light that threatens eye health. Blue light is the light with blue color detected in 400 ~ 500 nm among the visible light, which is exemplified by the light emitted from digital devices or smart phones. When the digital device is turned on at night, even though the screen is white in real, it seems as blue overall, which is a good example of the blue light. If a person is exposed on the blue light for a long time, the light stimulates the optic nerve to cause fatigue and other eye diseases.

The blue light has a strong energy and high penetrating power. Therefore, once the light passes through eyes as it is, it makes the retina lose focus, suggesting that a subject is not seen clearly. The chronic exposure to blue light, therefore, is a reason of aging and degeneration of the retina. The affect on human by blue light is exemplified by dry eye, ocular fatigue, failing of eyesight, various eye diseases, visual cell (retina) aging, macular degeneration, and insomnia caused by melatonin synthesis inhibition (Ganka Gakkai Zasshi. 2001 Oct;105(10):687-95; Archives of Ophthalmology 1992; 110:99-104; Review of Ophthalmology Oct 15 2003; 10(10)).

The retina is a transparent thin film which is located inner-most to the ocular wall and contacted with the vitreous body. The retina converts optical information of a subject into electrical signals to deliver the image through the optic nerve to the visual area of the brain, suggesting that the retina plays a role as the primary visual information system. The retina comprises more than a hundred million light-sensitive photoreceptor cells, more than a million optic neurons so called ganglion cells, and numbers of neurons that act as a wire to connect them together. So, the retina is the most sophisticated organ of those in human body. The macula lutea, the center part of the retina, distinguishes colors and objects and provides vision, which is composed of a photoreceptor cell layer comprising cone cells and a ganglion cell layer. This region is thin, in which the electrical signals of images in the light are converted into chemical signals which are delivered to the brain through the optic nerve. The other part except the macula lutea recognizes the periphery and is functioning mainly in the dark. Approximately 30% of the human brain cells are used to treat the visual information sent by the retina. Once the retina is aged or has a problem caused by external factors, visual acuity and visual field would be weakened slowly, which leads to visual impairment and at the worst to blindness. Retinal disease is mainly divided into three groups; retinal detachment wherein the neural retina is detached from the retinal pigment epithelium and thereby the retina is separated to the backside of the eyeball with causing visual impairment; peripheral retinal degeneration causing problems in the retinal peripheral tissues; and macular degeneration causing problems in the macula retinae. When the retina is detached from the pigment epithelium, it cannot receive optical information about the received image and is not getting nutrition supply from the choroid. So, neurons are not functioning properly. If this problem is neglected, permanent neurodeatrophia is caused, resulting in blindness. The major cause of blindness is visual impairment which is caused by retinal disease. Retinal disease is developed with aging and is caused by genetic reason or high myopia or trauma. Blindness is the second most frequent ophthalmic disease next to cataract. Three major ophthalmic diseases causing blindness are diabetic retinopathy, macular degeneration, and glaucoma. Retinal disease is not lethal. However, according to the increased senior population and advancement of industrialization and diet, retinal disease is rapidly increasing. Therefore, it is important and urgent to develop a composition for treating retinal disease based on herbal medicines that we can take instead of synthetic therapeutic drugs, in addition to surgical method.

Spirulina maxima is a kind of microalgae reproducing in salty alkali tropical area, for example in Lake Chad, Africa, and in Lake Texcoco, Mexico, etc. Spirulina maxima cell contains a large volume of chlorophyll and phycocyanin, by which it absorbs the sun's ray in order to grow by active carbon dioxide assimilation. Due to such pigments, the algae is blue-green so that it has been classified into the blue-green algae.

Since electron microscope was developed, the cellular structure of microorganism has been minutely identified. As a result, it was disclosed that the structure of green algae or brown algae was different from the structure of higher plants. That is, the structure of green algae is a eukaryote structure, which is equal to that of a higher plant. In the meantime, the structure of blue-green algae was identified as a prokaryote structure which was similar to that of bacteria. Since the early 1960s, some microbiologists have supported that blue-green algae is closer to bacteria than algae so that it needs to be included in Bacteriomycota. Today, this claim is accepted and therefore the blue-green algae above is now classified into the group of blue-green bacteria. However, in the industrial field, it is still called "micro-algae" as usual.

The name Spirulina maxima is originated from its spiral shape. Considering it has double-stranded DNA, it is a spiral bacterium belonging cyanobacteria characteristically in the middle of the animal and the plant. Spirulina maxima is an edible microorganism composed of 55 ~ 70% protein, 6 ~ 9% lipid, 15 ~ 20% carbohydrate, and other minor ingredients such as minerals, vitamins, fibers, and figments. Spirulina maxima contains not just high concentration of protein but also all of 8 essential amino acids. The fat included in this microorganism is mostly free-fatty acids (70 ~ 80%), which are exemplified by linoleic acid and γ-linolenic acid. Spirulina maxima has a low concentration of carbohydrate. However, it contains rhamnose and glycogen so that it can be absorbed without help of insulin, suggesting that it can be used as an energy source for diabetes patients. Native people have been collected this micro-algae for food. From the nutritional study, it was confirmed that this algae was composed of advantageous ingredients such as high concentration of protein including amino acids and other nutrients which are helpful for human body. The advantageous ingredients above are exemplified by Allophycocyanin, R-phycoerythin, and C-phycocyanin (Nanni B. et al., Microbiol Res. 2001; 156(3):259-66; Hangeul Donguibogam http://donguibogam.co.kr).

The present inventors tried to develop a composition for preventing and treating retinal disease. As a result, the inventors confirmed that Spirulina maxima extract had the effect of inhibiting cell death and A2E (pyridinium bis-retinoid) oxidized by blue light. Thereafter, the present inventors confirmed that the said Spirulina maxima extract and the major components of the same such as Allophycocyanin, R-phycoerthin, and C-phycocyanin could be useful as a pharmaceutical composition for preventing and treating retinal disease, leading to the completion of this invention.

### SUMMARY OF THE INVENTION

It is an object of the present invention to provide a pharmaceutical composition for preventing and treating retinal disease comprising Spirulina maxima extract and Allophycocyanin, R-phycoerythrin, or C-phycocyanin as an active ingredient, and a health food for improving retinal disease comprising the same.

To achieve the above object, the present invention provides a pharmaceutical composition for preventing and treating retinal disease comprising Spirulina maxima extract as an active ingredient.

The present invention also provides a heath functional food for preventing and improving retinal disease comprising Spirulina maxima extract as an active ingredient.

The present invention further provides a pharmaceutical composition for preventing and treating retinal disease comprising Allophycocyanin, R-phycoerythrin, or C-phycocyanin as an active ingredient.

In addition, the present invention provides a health functional food for preventing and improving retinal disease comprising Allophycocyanin, R-phycoerythrin, or C-phycocyanin as an active ingredient.

### ADVANTAGEOUS EFFECT

The Spirulina maxima extract of the present invention and Allophycocyanin (APC), R-phycoerythrin (R-PE), and C-phycocyanin (C-PC), which are components of the Spirulina maxima extract, show an effect of inhibiting cell death and A2E (pyridinium bis-retinoid) which is oxidized with a blue light, so that the present invention can be usefully applied as an active ingredient in a composition for preventing and treating retinal disease.

### BRIEF DESCRIPTION OF THE DRAWINGS

The application of the preferred embodiments of the present invention is best understood with reference to the accompanying drawings, wherein:
Figure 1 is a graph illustrating the inhibiting effect of Allophycocyanin (APC), R-phycoerythrin (R-PE), and C-phycocyanin (C-PC) on oxidized A2E (pyridinium bis-retinoid).
Figure 2 is a graph illustrating the cell protecting effect of Allophycocyanin, R-phycoerythrin, and C-phycocyanin from cell death caused by blue light in ARPE-19 cells containing A2E accumulated therein (post-treated with the sample).
   A2E: the cells not treated with blue light after A2E accumulation,
   A2E BL: the cells treated with blue light after A2E accumulation (negative control),
   APC: 25 **µg/Mℓ,**
   R-PE: 25 **µg/Mℓ,** and
   C-PC: 6.25, 12.5, 25, 50, and 100 **µg/Mℓ.**
Figure 3a is a graph illustrating the cell protecting effect of Allophycocyanin, R-phycoerythrin, and C-phycocyanin from cell death caused by blue light in ARPE-19 cells (preliminary experiment for Figure 3b).
   APC: 12.5, 25, and 40 **µg/Mℓ**,
   R-PE: 12.5, 25, and 40 **µg/Mℓ**, and
   C-PC: 12.5, 25, 50, 100, and 200 **µg/Mℓ**.
Figure 3b is a graph illustrating the cell protecting effect of Allophycocyanin, R-phycoerythrin, and C-phycocyanin from cell death caused by blue light in ARPE-19 cells (pre-treated with the sample).
   APC: 12.5 **µg/Mℓ**,
   R-PE: 12.5 **µg/Mℓ**, and
   C-PC: 25, 50, and 100 **µg/Mℓ**.
Figure 4 is a graph illustrating the cell protecting effect of Spirulina maxima extracts (products in Myanmar, Hawaii, and KIOST) from cell death in ARPE-19 cells containing A2E accumulated therein (post-treated with the sample).
Figure 5a is a graph illustrating the cell protecting effect of Spirulina maxima extracts (products in Myanmar, Hawaii, and KIOST) from cell death in ARPE-19 cells (preliminary experiment for Figure 5b).
   product in Myanmar: 125, 250, 500, 750, and 1000 **µg/Mℓ**,
   product in Hawaii: 250, 500, and 1000 **µg/Mℓ**, and
   product in KIOST: 250, 500, and 1000 **µg/Mℓ**.
Figure 5b is a graph illustrating the cell protecting effect of Spirulina maxima extract (product in KIOST) from cell death caused by blue light in ARPE-19 cells (pre-treated with the sample).
   product in KIOST: 62.5, 125, 250, and 500 **µg/Mℓ**.

### DESCRIPTION OF THE PREFERRED EMBODIMENTS

Hereinafter, the present invention is described in detail.

The present invention provides a pharmaceutical composition for preventing and treating retinal disease comprising Spirulina maxima extract as an active ingredient.

The said Spirulina maxima extract preferably contains one or more ingredients selected from the group consisting of Allophycocyanin (APC), R-phycoerythrin (R-PE), and C-phycocyanin (C-PC).

The Spirulina maxima extract is preferably prepared by the method comprising the following steps, but not always limited thereto:
1) extracting Spirulina maxima after adding an extraction solvent to Spirulina maxima;
2) filtering the extract prepared in step 1); and
3) concentrating the extract filtered in step 2) under reduced pressure, followed by drying thereof.

In the method above, the Spirulina maxima of step 1) can be cultivated or purchased.

The extraction solvent herein is preferably water, alcohol, or the mixture thereof. The said alcohol is preferably C₁ ~ C₂ lower alcohol. The lower alcohol herein is preferably ethanol or methanol. The extraction method is preferably high temperature extraction under reduced pressure, boiling extraction, reflux extraction, hot-water extraction, enfleurage, room temperature extraction, ultrasonic extraction, centrifugal extraction, or vapor extraction, but not always limited thereto. Particularly, the extraction solvent is added to Spirulina maxima at the ratio of 1 ~ 10 times the Spirulina maxima volume. The preferable temperature for the extraction is 30°C ~ 100°C, but not always limited thereto. The extraction hours are 2 ~ 48 hours, but not always limited thereto. The extraction is preferably repeated 2 ~ 5 times, but not always limited thereto.

In this method, concentration under reduced pressure in step 3) is preferably performed by using a vacuum concentrator or a vacuum rotary evaporator, but not always limited thereto. Drying herein is preferably performed by reduced-pressurized drying, vacuum drying, boiling drying, spray drying, or freeze drying, but not always limited thereto.

The Spirulina maxima extract has the effect of inhibiting A2E (pyridinium bis-retinoid) oxidized by blue light and can inhibit retinal cell death.

The retinal disease herein is one or more diseases selected from the group consisting of macular degeneration, glaucoma, Usher syndrome, Stargardt disease, Bardet-Biedl syndrome, Best disease, choroideremia, gyrate-atrophy, retinitis pigmentosa, macular degeneration, Leber congenital amaurosis (Leber's Hereditary Optic Neuropathy), BCM (blue-cone monochromacy), retinoschisis, ML (Malattia Leventinese), Oguchi disease, and Refsum disease.

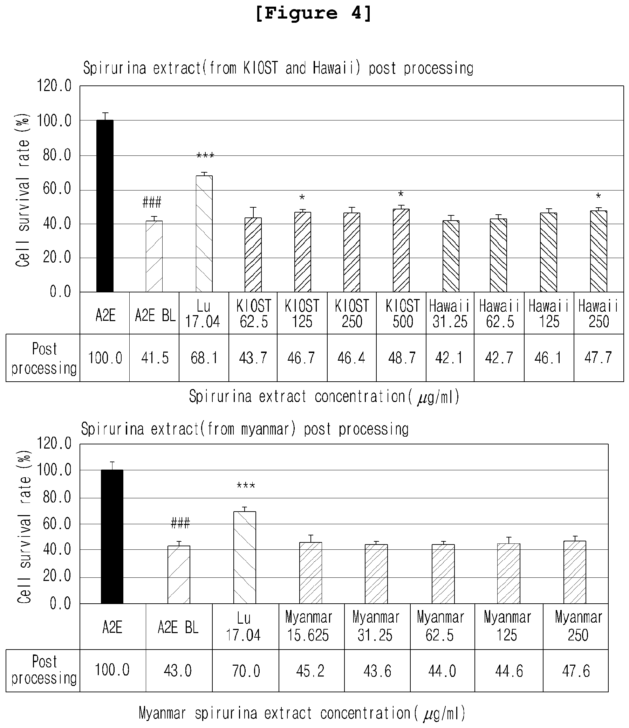

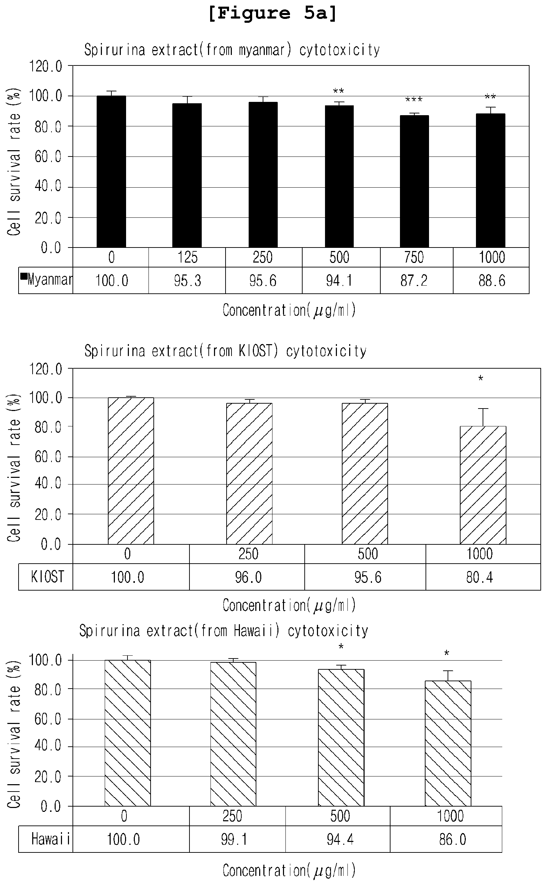

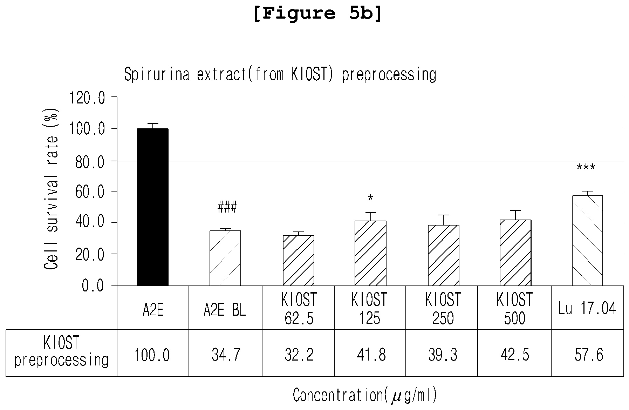

In a preferred embodiment of the present invention, the cell protecting effect of Spirulina maxima extracts (products in Myanmar, Hawaii, and KIOST) from cell death in ARPE-19 cells containing A2E accumulated therein was measured. As a result, the Spirulina maxima extracts prepared from the Spirulina maxima originated from Hawaii and KIOST exhibited the cell protecting effect dose-dependently. However, the Spirulina maxima extract prepared from the Spirulina maxima originated from Myanmar did not display statistically significant cell protecting effect (see Figure 4). Based on the KIOST originated Spirulina maxima extract dose-dependent retinal cell death preventing effect in ARPE-19 cells, obtained in Figure 5a, the cell protecting effect according to the inhibition of A2E accumulation and the inhibition of photooxidation was measured. As a result, survival rate (%) of the cells treated with the KIOST originated Spirulina maxima extract at the concentrations of 62.5, 125, 250, and 500 **µg/Mℓ** was increased respectively by 0%, 10.8%, 7.0%, and 11.9% (see Figure 5b).

Therefore, the Spirulina maxima extract (products in Hawaii and KIOST) had the cell protecting effect from cell death caused by blue light and had excellent cell protecting effect when photooxidation was inhibited, suggesting that the Spirulina maxima extract could be used as a pharmaceutical composition for preventing and treating retinal disease.

The pharmaceutical composition containing the extract of the present invention can include, in addition to the extract, one or more effective ingredients having the same or similar function to the extract.

The pharmaceutical composition of the present invention can additionally include a pharmaceutically acceptable additive, which is exemplified by starch, gelatinized starch, microcrystalline cellulose, lactose, povidone, colloidal silicon dioxide, calcium hydrogen phosphate, lactose, mannitol, taffy, Arabia rubber, pregelatinized starch, corn starch, cellulose powder, hydroxypropyl cellulose, Opadry, sodium carboxy methyl starch, carunauba wax, synthetic aluminum silicate, stearic acid, magnesium stearate, aluminum stearate, calcium stearate, white sugar, dextrose, sorbitol, talc, etc. The pharmaceutically acceptable additive herein is preferably added by 0.1 - 90 weight part to the pharmaceutical composition.

The pharmaceutical composition of the present invention can be administered orally or parenterally and be used in general forms of pharmaceutical formulation. The composition of the present invention can be prepared for oral or parenteral administration by mixing with generally used diluents or excipients such as fillers, extenders, binders, wetting agents, disintegrating agents and surfactant. Solid formulations for oral administration are tablets, pills, powders, granules and capsules. These solid formulations are prepared by mixing the extract of the invention with one or more suitable excipients such as starch, calcium carbonate, sucrose or lactose, gelatin, etc. Liquid formulations for oral administrations are suspensions, solutions, emulsions and syrups, and the above-mentioned formulations can contain various excipients such as wetting agents, sweeteners, aromatics and preservatives in addition to generally used simple diluents such as water and liquid paraffin. Formulations for parenteral administration are sterilized aqueous solutions, water-insoluble excipients, suspensions, emulsions, lyophilized preparations, suppositories and injections. Water insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerogelatin, etc.

The pharmaceutical composition of the present invention can be administered orally or parenterally and the parenteral administration includes skin external application, intraperitoneal injection, subcutaneous injection, intravenous injection, intramuscular injection and intrathoracic injection. The effective dose can be determined according to weight, age, gender, health condition, diet, administration frequency, administration method, excretion and severity of disease.

The effective dose of the composition of the present invention can be determined according to weight, age, gender, health condition, diet, administration frequency, administration method, excretion and severity of disease. The preferable dose is 0.0001 ~ 100 mg/kg per day, and more preferably 0.001 ~ 10 mg/kg per day, and administration frequency is preferably 1 ~ 6 times a day.

The pharmaceutical composition of the present invention can be administered alone or together with surgical operation, hormone therapy, chemo-therapy and biological regulators to prevent and treat retinal disease.

The present invention provides a pharmaceutical composition for preventing and treating retinal disease comprising one of the components selected from the group consisting of Allophycocyanin, R-phycoerythrin, and C-phycocyanin as an active ingredient.

The said Allophycocyanin, R-phycoerythrin, or C-phycocyanin has the effect of inhibiting A2E (pyridinium bis-retinoid) oxidized by blue light and can inhibit retinal cell death.

The retinal disease herein is one or more diseases selected from the group consisting of macular degeneration, glaucoma, Usher syndrome, Stargardt disease, Bardet-Biedl syndrome, Best disease, choroideremia, gyrate-atrophy, retinitis pigmentosa, macular degeneration, Leber congenital amaurosis (Leber's Hereditary Optic Neuropathy), BCM (blue-cone monochromacy), retinoschisis, ML (Malattia Leventinese), Oguchi disease, and Refsum disease.

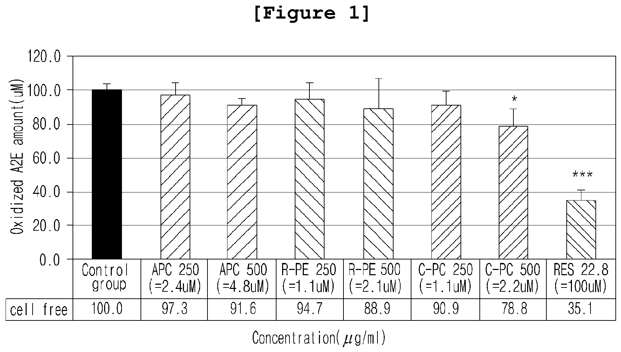

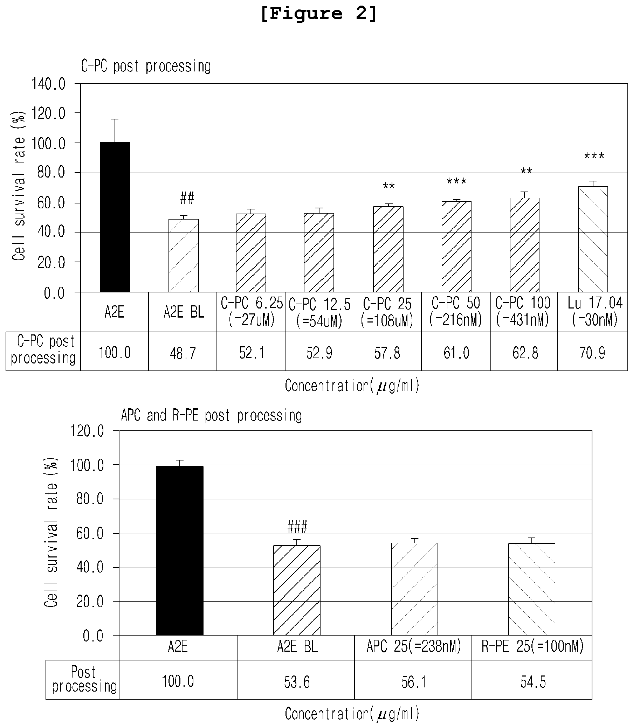

In a preferred embodiment of the present invention, the inventors measured the oxidation inhibiting effect of Allophycocyanin (APC), R-phycoerythrin (R-PE), and C-phycocyanin (C-PC) on pyridinium bis-retinoid (A2E) oxidized by blue light. As a result, the oxidation inhibiting effect of C-PC on A2E was statistically significant. In particular, the oxidation inhibiting effect of C-PC on A2E was the greatest, followed by R-PE and APC in that order (C-PC >R-PE > APC) (see Figure 1). The cell protecting effect of APC, R-PE, and C-PC against retinal cell death was investigated in ARPE-19 cells wherein A2E was accumulated. As a result, in the group treated with C-PC, the cell survival rate was increased dose-dependently by 6.7%, 8.1%, 17.8%, 23.9%, and 27.6%, while the cytotoxicity was not observed in the groups treated with APC and R-PE (see Figure 2).

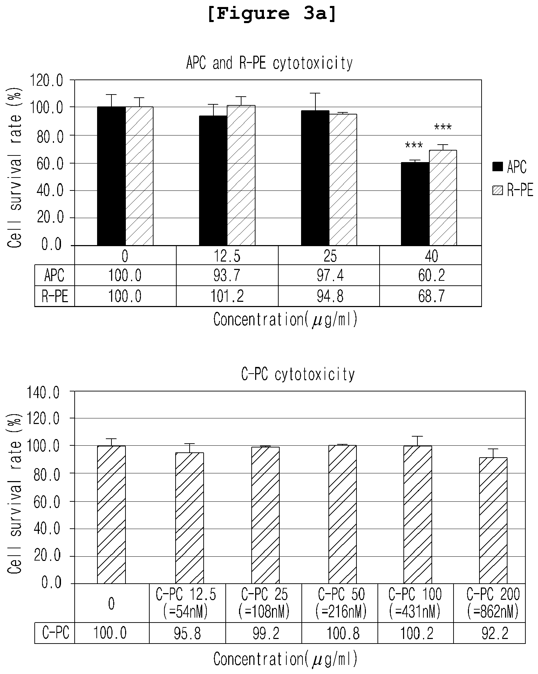

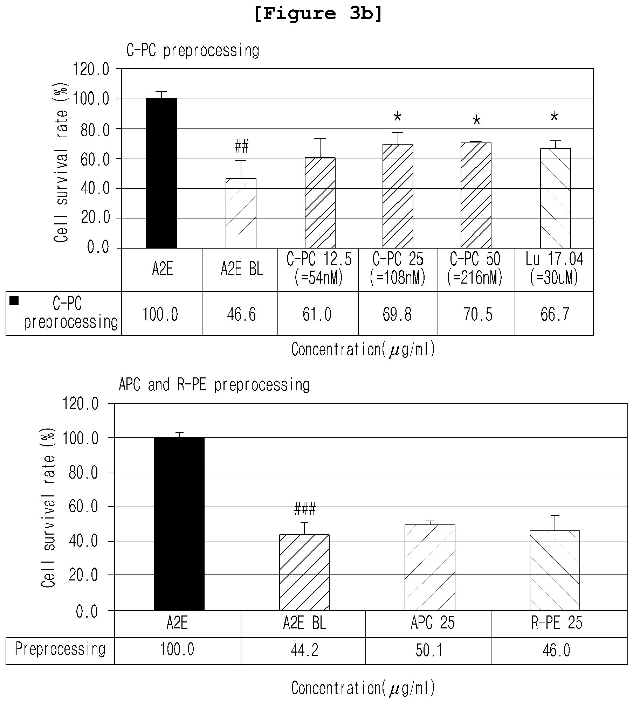

ARPE-19 cells were pre-treated with APC (25 **µg/Mℓ**), R-PE (25 **µg/Mℓ**), and C-PC (6.25, 12.5, 25, and 50 **µg/Mℓ**) according to the selected C-PC concentrations in Figure 3a. After confirming the accumulation of A2E, the cells were irradiated with blue light and then cell survival rate was investigated. In the group treated with C-PC, the cell survival rate was increased dose-dependently. However, in those groups treated with APC and R-PE, the cell protecting effect was not statistically significant (see Figure 3b).

Therefore, APC, R-PE, or C-PC was confirmed to have cell protecting effect against cell death caused by blue light and was excellent in cell protection by inhibiting photooxidation, so that APC, R-PE, or C-PC can be advantageously used as a pharmaceutical composition for preventing and treating retinal disease.

The present invention also provides a heath functional food for preventing and improving retinal disease comprising Spirulina maxima extract as an active ingredient.

The said Spirulina maxima extract has the effect of inhibiting A2E (pyridinium bis-retinoid) oxidized by blue light and can inhibit retinal cell death.

The retinal disease herein is one or more diseases selected from the group consisting of macular degeneration, glaucoma, Usher syndrome, Stargardt disease, Bardet-Biedl syndrome, Best disease, choroideremia, gyrate-atrophy, retinitis pigmentosa, macular degeneration, Leber congenital amaurosis (Leber's Hereditary Optic Neuropathy), BCM (blue-cone monochromacy), retinoschisis, ML (Malattia Leventinese), Oguchi disease, and Refsum disease.

Therefore, the Spirulina maxima extract was confirmed to have cell protecting effect against cell death caused by blue light and was excellent in cell protection by inhibiting photooxidation, so that the Spirulina maxima extract can be advantageously used as a heath functional food for preventing and improving retinal disease.

In addition, the present invention provides a method for treating or preventing retinal disease containing the step of administering an effective dose of Spirulina maxima extract to a subject having retinal disease or a normal subject.

The present invention also provides a Spirulina maxima extract for the drug for preventing and treating retinal disease or for the health food for preventing and improving retinal disease.

The present invention also provides a health functional food for preventing and improving retinal disease comprising one or at least two of those active ingredients selected from the group consisting of Allophycocyanin, R-phycoerythrin, and C-phycocyanin.

The said Allophycocyanin, R-phycoerythrin, or C-phycocyanin has the effect of inhibiting A2E (pyridinium bis-retinoid) oxidized by blue light and can inhibit retinal cell death.

Therefore, APC, R-PE, or C-PC was confirmed to have cell protecting effect against cell death caused by blue light and was excellent in cell protection by inhibiting photooxidation, so that APC, R-PE, or C-PC can be advantageously used as a health functional food for preventing and improving retinal disease.

In addition, the present invention provides a method for treating or preventing retinal disease containing the step of administering an effective dose of one or at least two of those active ingredients selected from the group consisting of Allophycocyanin, R-phycoerythrin, and C-phycocyanin to a subject having retinal disease or a normal subject.

The present invention also provides one or at least two of those active ingredients selected from the group consisting of Allophycocyanin, R-phycoerythrin, and C-phycocyanin for the drug for preventing and treating retinal disease or for the health food for preventing and improving retinal disease.

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Example 1: Preparation of Spirulina maxima extract

Spirulina maxima (Korea Marine Microalgae Culture Center Accession No: KMMCC-1057) was provided from Korea Marine Microalgae Culture Center, Department of Marine Bio-materials & Aquaculture, Pukyong National University, Korea.

Particularly, the cultured Spirulina maxima was centrifuged in multi-tube carrier refrigerated centrifuge (Vision Scientific CO. Ltd) at 3000 rpm for 25 minutes. The separated cells were washed simply with 1.0% NaCl solution, followed by centrifugation again. The obtained cells were freeze-dried, which were used as a sample for phycocyanin extraction. The extraction was performed as follows: 10 **Mℓ** of 0.1 M phosphate buffer (pH 7.0) was added to 40 **mg** of the freeze-dried sample, followed by vortexing for 15 minutes. Supernatant was obtained by centrifugation (3, 500 rpm, 5 minutes), which was used as a Spirulina maxima extract.

### Example 2: Preparation of Allophycocyanin, R-phycoerythrin, and C-phycocyanin

Allophycocyanin (A7472), R-phycoerythrin (P6161), and C-phycocyanin were purchased from Sigma-Aldrich, which were prepared at the concentrations of 4 **mg/Mℓ**, 10 **mg/Mℓ**, and 1 **mg/Mℓ**, respectively.

### Example 3: Cell culture

Human adult ARPE cells (ARPE-19: catalog no. CRL-2302) used for the experiment and analysis in this invention were distributed from Vision Science Research Center, College of Medicine, The Catholic University of Korea. The ARPE cells above were cultured in DMEM supplemented with 10% FBS, 100 U/**Mℓ** of penicillin, and 100 **mg/Mℓ** of streptomycin in a 5% CO₂, 37°C incubator. The cells were inoculated in a 6-well plate at the density of 5×10⁴ cells/well for further experiment.

### Example 3: A2E synthesis

A2E (pyridinium bis-retinoid) used for the experiment and analysis in this invention was synthesized as follows: all-trans-retinal dissolved in ethanol was mixed with ethanol amine (molar ratio: 2:1). Acetic acid was added to the mixture in the dark room, followed by reaction for 2 days. The mixture was vacuum-concentrated at 40°C, followed by purification using silica gel column chromatography. The synthesized A2E was dissolved in DMSO (Dimethyl Sulfoxide) at the stock concentration of 20 mM, which was stored at -20°C.

### Experimental Example 1: Oxidation inhibition effect of Allophycocyanin, R-phycoerythrin, and C-phycocyanin on pyridinium bis-retinoid (A2E) oxidized by blue light

The following experiment was performed to measure the oxidation inhibition effect of Allophycocyanin (APC), R-phycoerythrin (R-PE), and C-phycocyanin (C-PC) on retinal A2E oxidation.

Particularly, 20 **µℓ** of A2E (final concentration: 100 uM) was dissolved in 160 **µℓ** of PBS containing 0.01% DMSO, and the mixed solution was distributed in a 96-well (180**µℓ**/well). Each sample (control or APC, R-PE, and C-PC obtained from Spirulina maxima extract) was added (respectively 250 and 500 **µg/Mℓ** at the final concentration) to the plate (20 **µℓ**/well). OD₄₃₀ (430 nm: A2E absorption wavelength) was measured with ELISA microplate reader. The plate was irradiated with blue light at the energy strength of 2.01 J/cm², followed by measurement of OD again by the same manner as described above. The measured OD value was converted into the concentration by using A2E standard curve and the concentration of the oxidized A2E was calculated by the difference in concentration before and after the blue light irradiation.

As a result, as shown in Figure 1, when C-PC was treated to the cells (250 and 500 **µg/Mℓ**), the oxidized A2E was reduced 9.1% and 21.2% by the control (CTL, 100%), from which it was confirmed that C-PC had a statistically significant A2E oxidation inhibition effect dose-dependently. In the meantime, when APC was treated to the cells (250 and 500 **µg/Mℓ**), the oxidized A2E was reduced 2.7% and 8.4% by the control. When R-PE was treated to the cells (250 and 500 **µg/Mℓ**), the oxidized A2E was reduced 5.3% and 11.1% by the control. Therefore, it was confirmed that APC and R-PE both had the A2E oxidation inhibition effect, which was though not as great as that of C-PC. So, the A2E oxidation inhibition effect was decreased in the following order: C-PC>R-PE>APC (Figure 1).

### Experimental Example 2: Cell protection effect of APC, R-PE, and C-PC against retinal cell death in ARPE-19 cells having A2E accumulated therein (sample post-treatment system)

The following experiment was performed to investigate the cell protection effect of Allophycocyanin (APC), R-phycoerythrin (R-PE), and C-phycocyanin (C-PC) on retinal A2E oxidation.

Particularly, ARPE-19 cells were distributed in a 24-well plate at the density of 2x10⁴ cells/well, followed by accumulation of A2E (20 **µℓ**) for 7 days (final concentration: 10 uM, three times of treatment). According to the result of Figure 1, those three substances, APC (25 **µg/Mℓ**), R-PE (25 **µg/Mℓ**), and C-PC (6.25, 12.5, 25, 50, and 100 **µg/Mℓ**) were treated to the cells twice for 3 days. The cells were irradiated with blue light (4.02 J/cm²), followed by culture for 24 hours. Cell survival rate was measured by MTT assay. MTT assay is established based on the principal that yellow tetrazolium salt (MTT) reacts to reductase in mitochondria in a living cell to form purple formazan crystals. That is, as the population of live cells increases, the production of formazan crystals increases, resulting in the increased OD.

In MTT assay, DMEM containing 0.5 **mg/Mℓ** of MTT was added to the cells, and light was blocked, followed by culture in a 37°C incubator for 4 hours. Upon completion of the reaction, the cells were fully dissolved in 1 **Mℓ** of DMSO. OD₅₄₀ was measured with ELISA microplate reader. The cell survival rate was presented with % by the cell survival rate of the cell group (normal control; A2E) had A2E accumulated but not irradiated with blue light.

As a result, as shown in Figure 2, there was a significant difference between the group having A2E accumulated but not irradiated with blue light (A2E) and the group having A2E accumulated and irradiated with blue light (negative control: A2E BL). Compared with the cell survival rate of the negative control A2E (100%), the cell survival rate of each group treated with those three substances respectively (APC: 25 **µg/Mℓ**, R-PE: 25 **µg/Mℓ**, C-PC: 6.25, 12.5, 25, 50, and 100 **µg/Mℓ**) was increased as high as 6.7%, 8.1%, 17.8%, 23.9%, and 27.6%. On the other hand, in those groups treated with APC and R-PE at the concentration of 25 **µg/Mℓ**, which was the highest concentration that was free from cytotoxicity, the cell protection effect was not significant statistically (Figure 2).

### Experimental Example 3: Cell protection effect of Allophycocyanin, R-phycoerythrin, and C-phycocyanin from retinal cell death by the inhibition of A2E accumulation and the inhibition of photooxidation (sample pre-treatment system)

The following experiment was performed to investigate cell protection effect of Allophycocyanin (APC), R-phycoerythrin (R-PE), and C-phycocyanin (C-PC) on A2E accumulation and retinal A2E oxidation.

In the preliminary experiment (Figure 3a), the cytotoxicity caused by 0 ~ 40 **µg/Mℓ** of APC or R-PE and 0 ~ 200 **µg/Mℓ** of C-PC was investigated and the concentrations displaying the effect on cytotoxicity were selected.

Particularly, ARPE-19 cells were distributed in a 24-well plate at the density of 2x10⁴ cells/well, which were treated with the three substances (final conc., APC: 25 **µg/Mℓ**, R-PE: 25 **µg/Mℓ**, C-PC: 6.25, 12.5, 25, and 50 **µg/Mℓ**) on day 1, on day 4, and on day 7, three times in total. To accumulate A2E in cells, the cells were treated with A2E at the final concentration of 10 uM on day 2, on day 5, and on day 8, three times for 7 days, which were then irradiated with blue light (4.02 J/cm²), followed by culture for 24 hours. Then, the cell survival rate was measured by MTT assay. According to MTT assay method, DMEM containing 0.5 **mg/Mℓ** of MTT was added to the cells, and light was blocked, followed by reaction in a 37°C incubator for 4 hours. Upon completion of the reaction, the cells were fully dissolved in 1 **Mℓ** of DMSO. Then, OD₅₄₀ was measured with ELISA microplate reader. Cell survival rate was calculated by comparing that of the cell group having A2E accumulated but not irradiated with blue light and presented as % by that.

As a result, as shown in Figure 3b, there was a statistically significant difference between the group having A2E accumulated and not-irradiated with blue light (A2E) and the group having A2E accumulated and irradiated with blue light (negative control: A2E BL). The cell survival rate of the group treated with C-PC at the concentrations of 12.5, 25, and 50 **µg/Mℓ** was increased 26.9%, 43.4%, and 44.8% respectively by the cell survival rate of the negative control A2E BL. In the meantime, in the groups treated with APC and R-PE, the cell protection effect was not so significant at the concentration of 25 **µg/Mℓ** that was the highest concentration not causing cytotoxicity (Figure 3b).

### Experimental Example 4: Cell protection effect of Spirulina maxima extract according to the origins on retinal cell death in ARPE-19 cells having A2E accumulated (sample post-treatment system)

The following experiment was performed to investigate cell protection effect of Spirulina maxima extract according to the origins (Myanmar, Hawaii, and KIOST) on retinal A2E oxidation.

Particularly, ARPE-19 cells were distributed in a 24-well plate at the density of 2x10⁴ cells/well, and A2E was accumulated therein for 7 days by the same manner as described in Experimental Example 3 (final conc., 10 uM, three times). Then, the cells were treated with the three substances having different origins as follows: treated with the Myanmar originated Spirulina maxima extract at the final concentrations of 15.625, 31.25, 62.5, 125, and 250 **µg/Mℓ**, with the Hawaii originated Spirulina maxima extract at the final concentrations of 31.25, 62.5, 125, and 250 **µg/Mℓ**, and with the KIOST originated Spirulina maxima extract at the final concentrations of 62.5. 125, 250, and 10 **µg/Mℓ**, followed by irradiation with blue light (4.02 J/cm²). The cells were cultured for 24 hours. According to MTT assay method, DMEM containing 0.5 **mg/Mℓ** of MTT was added to the cells, and light was blocked, followed by reaction in a 37°C incubator for 4 hours. Upon completion of the reaction, the cells were fully dissolved in 1 **Mℓ** of DMSO, and OD₅₄₀ was measured with ELISA microplate reader. Cell survival rate was presented as % by the cell survival rate of the cells having A2E accumulated but not irradiated with blue light.

As a result, as shown in Figure 4, there was a statistically significant difference in the cell survival rate between the group having A2E accumulated but not irradiated with blue light (A2E) and the group having A2E accumulated and irradiated with blue light (negative control: A2E BL). The cell survival rate of each group treated respectively with three different extracts having different origins, precisely treated with the Myanmar originated Spirulina maxima extract at the concentrations of 15.625, 31.25, 62.5, 125, and 250 **µg/Mℓ**, with the Hawaii originated Spirulina maxima extract at the concentrations of 31.25, 62.5, 125, and 250 **µg/Mℓ**, and with the KIOST originated Spirulina maxima extract at the concentrations of 62.5, 125, 250, and 500 **µg/Mℓ**, was compared with the cell survival rate (100%) of the negative control. As a result, the cell survival rate of the group treated with the Hawaii originated Spirulina maxima extract was increased 1.0%, 2.0%, 7.9%, and 10.6%, respectively. In the meantime, the cell survival rate of the group treated with the KIOST originated Spirulina maxima extract was increased 3.8%, 8.9%, 8.4%, and 12.4%, respectively. Therefore, it was confirmed that the cell protection effect of the Hawaii originated Spirulina maxima extract was equal to that of the KIOST originated Spirulina maxima extract. On the other hand, in the group treated with the Myanmar originated Spirulina maxima extract, the cell protection effect was not statistically significant at any concentration (Figure 4).

### Experimental Example 5: Cell protection effect of KIOST originated Spirulina maxima extract on retinal cell death in ARPE-19 cells according to the inhibition of A2E accumulation and photooxidation (sample pre-treatment system)

The following experiment was performed to investigate cell protection effect of the KIOST originated Spirulina maxima extract on A2E accumulation and retinal A2E oxidation.

In the preliminary experiment (Figure 5a), the concentration that exhibited effect on cytotoxicity was selected after treating cells with 1 ~ 1000 **µg/Mℓ** of the KIOST originated Spirulina maxima extract. Particularly, ARPE-19 cells were distributed in a 24-well plate at the density of 2x10⁴ cell/well. The cells were treated with the extract at the final concentrations determined in Figure 5a (62.5, 125, 250, and 500 **µg/Mℓ**) on day 1, on day 4, and on day 7, three times in total. To make A2E deposited therein, the cells were treated with A2E at the concentration of 10 uM for 7 days three times respectively on day 2, day 5, and day 8. To accumulate A2E in cells, the cells were treated with A2E at the final concentration of 10 uM on day 2, on day 5, and on day 8, three times for 7 days, which were then irradiated with blue light (4.02 J/cm²), followed by culture for 24 hours. According to MTT assay method, DMEM containing 0.5 **mg/Mℓ** of MTT was added to the cells, and light was blocked, followed by reaction in a 37°C incubator for 4 hours. Upon completion of the reaction, the cells were fully dissolved in 1 **Mℓ** of DMSO. Then, OD₅₄₀ was measured with ELISA microplate reader. Cell survival rate was calculated by comparing that of the cell group having A2E accumulated but not irradiated with blue light and presented as % by that.

As a result, as shown in Figure 5b, there was a statistically significant difference in the cell survival rate between the group having A2E accumulated but not irradiated with blue light (A2E) and the group having A2E accumulated and irradiated with blue light (negative control: A2E BL). The cell survival rate of the cells treated with the KIOST originated Spirulina maxima extract at the concentrations of 62.5, 125, 250, and 500 **µg/Mℓ** was increased respectively by 0%, 10.8%, 7.0%, and 11.9% by that of A2E BL (100%) (Figure 5b).

## Claims

1. A pharmaceutical composition for preventing and treating retinal disease comprising Spirulina maxima extract as an active ingredient.

2. The pharmaceutical composition for preventing and treating retinal disease according to claim 1, wherein the Spirulina maxima extract characteristically contains one or more ingredients selected from the group consisting of Allophycocyanin (APC), R-phycoerythrin (R-PE), and C-phycocyanin (C-PC).

3. The pharmaceutical composition for preventing and treating retinal disease according to claim 1, wherein the Spirulina maxima extract is extracted by centrifugal extraction, solvent extraction, or ultrasonic extraction.

4. The pharmaceutical composition for preventing and treating retinal disease according to claim 1, wherein the Spirulina maxima extract characteristically exhibits the effect of inhibiting pyridinium bis-retinoid (A2E) oxidized by blue light.

5. The pharmaceutical composition for preventing and treating retinal disease according to claim 1, wherein the retinal disease is selected from the group consisting of macular degeneration, glaucoma, Usher syndrome, Stargardt disease, Bardet-Biedl syndrome, Best disease, choroideremia, gyrate-atrophy, retinitis pigmentosa, macular degeneration, Leber congenital amaurosis (Leber's Hereditary Optic Neuropathy), BCM (blue-cone monochromacy), retinoschisis, ML (Malattia Leventinese), Oguchi disease, and Refsum disease.

6. The pharmaceutical composition for preventing and treating retinal disease according to claim 1, wherein the Spirulina maxima extract characteristically inhibits retinal cell death.

7. A health functional food for preventing and improving retinal disease comprising Spirulina maxima extract as an active ingredient.

8. The health functional food for preventing and improving retinal disease according to claim 7, wherein the Spirulina maxima extract is extracted by centrifugal extraction, solvent extraction, or ultrasonic extraction.

9. A method for treating retinal disease containing the step of administering an effective dose of the Spirulina maxima extract to a subject having retinal disease.

10. A method for preventing retinal disease containing the step of administering an effective dose of the Spirulina maxima extract to a subject.

11. A Spirulina maxima extract for the preparation of a drug for preventing and treating retinal disease.

12. A Spirulina maxima extract for the preparation of a health functional food for preventing and improving retinal disease.

13. A pharmaceutical composition for preventing and treating retinal disease comprising one or more active ingredients selected from the group consisting of Allophycocyanin (APC), R-phycoerythrin (R-PE), and C-phycocyanin (C-PC).

14. The pharmaceutical composition for preventing and treating retinal disease according to claim 13, wherein the Allophycocyanin, R-phycoerythrin, or C-phycocyanin has the effect of inhibiting pyridinium bis-retinoid (A2E) oxidized by blue light.

15. The pharmaceutical composition for preventing and treating retinal disease according to claim 13, wherein the Allophycocyanin, R-phycoerythrin, or C-phycocyanin characteristically inhibits retinal cell death.

16. A health functional food for preventing and improving retinal disease comprising one or at least two of those active ingredients selected from the group consisting of Allophycocyanin, R-phycoerythrin, and C-phycocyanin.

17. A method for treating retinal disease containing the step of administering an effective dose of one or at least two of those active ingredients selected from the group consisting of Allophycocyanin, R-phycoerythrin, and C-phycocyanin to a subject having retinal disease.

18. A method for preventing retinal disease containing the step of administering an effective dose of one or at least two of those active ingredients selected from the group consisting of Allophycocyanin, R-phycoerythrin, and C-phycocyanin to a subject.

19. A use of Allophycocyanin, R-phycoerythrin, and C-phycocyanin for the preparation of a drug for preventing and treating retinal disease.

20. A use of Allophycocyanin, R-phycoerythrin, and C-phycocyanin for the preparation of a health functional food for preventing and improving retinal disease.
